Europäisches Patentamt

European Patent Office

Office européen des brevets

(19)

(11) Publication number: **0 029 697**
**A1**

(12) # EUROPEAN PATENT APPLICATION

(21) Application number: **80304127.6**

(22) Date of filing: **18.11.80**

(51) Int. Cl.³: **A 61 K 31/425**
**//C07D277/18**

(30) Priority: **20.11.79 EP 79810161**

(43) Date of publication of application:
**03.06.81 Bulletin 81/22**

(84) Designated Contracting States:
**BE CH DE FR GB IT LI LU NL SE**

(71) Applicant: **Gosalvez, Mario Gosalvez**
**Caleruega 21 7a Pinar de Chamartin**
**Madrid(ES)**

(72) Inventor: **Gosalvez, Mario Gosalvez**
**Caleruega 21 7a Pinar de Chamartin**
**Madrid(ES)**

(74) Representative: **Fisher, Bernard et al,**
**Raworth, Moss & Cook 36 Sydenham Road**
**Croydon Surrey CR0 2EF(GB)**

(54) **Pharmaceutical compositions for the treatment of neoplastic diseases and process therefor.**

(57) A pharmaceutical composition for the treatment of neoplastic diseases containing 2-amino-2-thiazoline or a physiologically acceptable salt thereof. The 2-amino-2-thiazoline or a physiologically acceptable salt thereof can be contained in an injection solution in lyophilized form. The composition may be in solid or powder form and contain the 2-amino-2-thiazoline or a physiologically acceptable salt thereof in lyophilized form.

EP 0 029 697 A1

Pharmaceutical Compositions for the Treatment of
Neoplastic Diseases and Process therefor

This invention relates to chemotherapeutic agents
for the treatment of malignant tumors or other cancerous
conditions.

The volume of research directed towards understanding the causes and finding a cure for cancer has greatly
increased in the last ten years as a result of increased
public concern with the disease. An outgrowth of this
research has been the discovery and utilization of a large
number of anti-cancer drugs. A common feature of these
new drugs is their cytotoxic activity, i.e., they act
by killing the cell.

The use of cytotoxic drugs has been a typical first
step in the chemotherapeutic treatment of diseases caused
by an aggressive cell. This was the case, for example,
in the early treatment of tuberculosis, a disease which
has now been largely conquered but which was once as dreaded as cancer is today.

As indicated above, cytotoxic agents act by killing
cells. Unfortunately, they are not selective and attack
healthy or normal cells as well as foreign or diseased
ones. Nevertheless, using such agents, skilled specialists
were able to add 10-15 years to the life expectancy of
persons afflicted with tuberculosis. However, a price had to be paid as the patient suffered terribly from the treatments with these toxic agents. For example, vomiting, hair loss, loss of teeth and other effects had to be endured.

In view of the great shortcomings associated with the use of cytotoxic agents, it could not be said that tuberculosis was curable until non-cytotoxic antibiotic drugs capable of selectively attacking the tuberculosis bacillus became available. Such drugs could be readily used by even general practitioners in relatively simple schemes of treatment. As a result, tuberculosis today has largely been eliminated.

The chemotherapy of cancer is presently limited to cytotoxic drugs that kill normal cells even as they attack the tumor cells. For certain kinds of cancer, skilled specialists using these agents, typically in combinations, have been able to maintain patients relatively free from disease for periods of ten to fifteen years. However, the patient undergoing treatment with these drugs has had to endure great suffering because the cytotoxic agents are severely toxic. Furthermore, the complexity of the treatments preclude their successful use by all but the most highly skilled physicians having access to the best medical facilities. Obviously, many of those who are afflicted with cancer, particularly in underdeveloped countries, do not have access to this kind of treatment. As a result, no one is yet prepared to characterize the limited success achieved using these drugs representing a cure for the cancer involved. That must await the availability of non-toxic drugs that can be readily employed by any physician.

In addition to leading to the discovery of additional cytotoxic agents to be added to the chemical arsenal of anti-cancer drugs, the increased research of recent years has lead to discoveries which are laying the groundwork for a fundamental understanding of the nature of cancer. Out of that research has emerged the concept that the main biological and biochemical differences between a cancer cell and a normal cell are found in the plasma membrane and the cytoskeletal system of contractile microfilaments which connect the plasma membrane with the cell nucleus. It has been postulated that the process by which a normal cell is transformed by a virus or chemical carcinogen to a tumor cell involves a disorganization of the cytoskeletal system of contractile filaments.

Another discovery has been that cancerous cells lack the property of "contact inhibition" that is exhibited by normal cells. Contact inhibition is the property of normal cells when in contact that is manifest by the cessation of cell movement, cell growth and cell division. Because they lack this property, cancer cells when in contact continue to grow and divide. As a result, the cancer cells are highly invasive of adjacent normal tissue. Furthermore, the lack of contact inhibition can result in metastasis, the occurrence of secondary tumors at other body locations caused by tumor cells released into the blood stream.

An important recent discovery has been the observation that tumor cells can be caused to undergo a "reverse transformation" in which the cells take on a morphology akin to that of normal cells in that the cytoskeletal system of contractile filaments in the cells is reorganized and the property of contact inhibition is restored by treatment with dibutyryl cyclic AMP (adenosine monophosphate). Cyclic AMP is an agent that naturally occurs in low concentration in cells. However, it serves many cellular functions and, therefore, cannot be administered to humans in a dosage sufficient to restore contact inhibition in cancer cells. This observation has spurred the hope that a specific agent can be found which will induce reverse transformation in cancer cells and restore contact inhibition. Such an agent would be expected to arrest tumor growth and perhaps lead to a slow regression of the tumor by gradual replacement of the tumorous cells with healthy ones.

In view of the state of the art summarized above, one object of the present invention is to provide pharmaceutical compositions for the treatment of cancer in mammals, and particularly for the treatment of cancer in humans.

In accordance with the present invention a dosage of 2-amino-2-thiazoline or a pharmaceutically acceptable salt thereof to an afflicted subject, e.g., a mammal having a malignant tumor or other cancerous condition, in an amount effective to arrest or retard the growth of the tumor, causes reverse transformation of the tumor cells and/or restores the property of contact inhibition. The administration of the drugs may be oral or parenteral, e.g. intramuscular or intravenous.

2-Amino-2-thiazoline is a known compound that has been described in the literature to be pharmaceutically active. However, up to now it was unknown that 2-amino-2-thiazoline and physiologically acceptable salts thereof are useful in the chemotherapy of neoplastic diseases and malignant tumors of the kind that afflict humans and other mammals, particularly in the cure of human cancer. A preferred species of cancer to be treated in accordance with the present invention is bladder cancer. Any salt of 2-amino-2-thiazoline with an organic or inorganic acid that is pharmaceutically acceptable in an effective amount may be used and the free base can also be used if it is free of polymers. In that regard, the salts of 2-amino-2-thiazoline which are most useful in this invention are the salts with hydrohalic acids, especially the hydrochloride.

In a specific embodiment of this invention the active agents are used as a lyophilized powder that is readily soluble in aqueous media and, therefore, well suited for formulations for oral or parenteral administration.

To conveniently obtain a salt as a lyophilized powder, an aqueous solution of 2-amino-2-thiazoline is neutralized to a pH of about 7 with a suitable acid. The neutralization may be conducted in the presence of mannitol, lactose, glycine, or other suitable supports for lyophilization. The neutralization should be effected as quickly as possible and the solution should be protected from light during the process. After filtration, the solution is frozen and lyophilized using conventional freeze drying techniques. The resulting dry powder can be reconstituted with water for parenteral injection or used in the preparation of pills for oral administration. On the other hand the pure free base can be obtained from the salts in accordance with known methods, e.g. by reaction with an equivalent amount of a strong base.

Tests on mice, rats and dogs have shown 2-amino-2-thiazoline and 2-amino-2-thiazoline hydrochloride to be completely non-toxic in doses up to 100 mg/kg of body weight. 2-amino-2-thiazoline and 2-amino-2-thiazoline hydrochloride are also non-toxic to humans in doses up to at least 100 mg/kg. Therefore, the dosage administered can vary over a wide range, without concern that undesirable side-effects will result from large dosages.

An effective dosage in the process of the present invention may vary, for example, with the kind of malignancy involved. Typically, a dosage of from about 1-50 mg/kg of body weight is administered daily for a sufficient time to arrest or retard the growth of the tumor, to induce reverse transformation in malignant tumor cells and to restore the property of contact inhibition to those cells. Preferably, the daily dosage rate will be in the range of from about 5-20 mg/kg of body weight.

While daily administration is preferred, it will be understood that other treatment schedules on other than a daily basis may be suitable in specific situations. As already indicated, 2-amino-2-thiazoline or its salts can be administered orally, preferably in pill form, or intramuscularly or intravenously, by injection of an aqueous solution. A suitable concentration of the injection solutions is 1-100 ml/kg, preferably 25-50 mg/ml.

The efficacy of 2-amino-2-thiazoline hydrochloride and its salts as non-cytotoxic agents for treating malignancies, is shown by the following experiments.

EXPERIMENTAL RESULTS

Restoration of Contact Inhibition in Colonies of Hela Cells using 2-Amino-2-thiazoline

Hela cells, a type of cell derived from human uterine tumor, were seeded in Eagle culture flasks at the concentration of 50 cells/cm$^2$. The cells were allowed to develop colonies during a period of 10 days with two changes of the medium on the second and sixth day. A tissue culture (minimum essential medium of Eagle with 10% calf serum) comprising amino acids, vitamins and glucose was employed.

2-Amino-2-thiazoline, obtained by treatment of the hydrochloride with a molar equivalent of sodium hydroxide, was added to the medium at varying concentrations on both the second day and the sixth day, after the initiation of the culture. A total of 3 flasks containing the cultured colonies were used for each concentration of 2-amino-2-thiazoline employed. On the tenth day, the cells were fixed in neutral formol and were stained with hematoxyline. A total of 500 colonies of the same size in each flask were counted at random and the percentage of colonies with contact inhibition (planar morphology) or without contact inhibition (pyrimidal or acuminated morphology) were noted. The results of these experiments are shown in Table I. As can be seen from that table, in those cultures which developed in the absence of the compound, about 50% of the colonies developed without contact inhibition. Those cultures treated with 2-amino-2-thiazoline showed an increased amount of colonies with contact inhibition. At the maximum dosage, 100% of the colonies showed contact inhibition. This experiment demonstrates that 2-amino-2-thiazoline is able to restore contact inhibition in human tumor cells.

Effect of 2-Amino-2-thiazoline Hydrochloride on Tumors in Humans

Clinical trials with 2-amino-2-thiazoline hydrochloride have been conducted on 9 patients suffering from advanced metastastic bladder cancer, not amenable to any other treatment. The results of these trials are shown in Table II.

Only patients with measurable and progressing bladder tumors and lung metastases the growth rate of which was measured weekly in X-ray plates for serveral weeks before beginning the treatment were admitted to the trial. The development of the bladder tumors was measured by cytoscopy after biopsy.

## TABLE I

| 2-Amino-2-thiazoline [μMol] | Colonies with contact inhibition [%] | Colonies without contact inhibition [%] | Total number of colonies [%] |
|---|---|---|---|
| 0 | 58 | 42 | 100 |
| 1 | 58 | 42 | 100 |
| 2 | 59 | 41 | 100 |
| 5 | 75 | 25 | 85 |
| 10 | 86 | 14 | 75 |
| 20 | 100 | 0 | 45 |

## TABLE II

### Clinical Study of the Effect of 2-Amino-2-thiazoline·HCl in Advanced Cancer

| Diagnosis | Measurable lesion | Total dose received | Toxicity | Response |
|---|---|---|---|---|
| bladder carcinoma | lung and bladder | 2.5 gr | none | complete remission |
| bladder carcinoma | lung and bladder | 3.2 gr | none | partial remission |
| bladder carcinoma | lung and bladder | 2.8 gr | none | complete remission |
| bladder carcinoma | bladder | 3.7 gr | none | stabilization |
| bladder carcinoma | lung and bladder | 4.9 gr | none | stabilization |
| bladder carcinoma | bladder | 12 gr | none | complete remission |
| bladder carcinoma | lung and bladder | 9.6 gr | none | complete remission |
| bladder carcinoma | lung and bladder | 11.8 gr | none | stabilization |
| bladder carcinoma | lung and bladder | 10.8 gr | none | stabilization |

This kind of patient was selected in order to determine whether administration of 2-amino-2-thiazoline would cause an arrest of tumor or a slow-down in the rate of growth with eventual regression of tumor size. It was anticipated that tumor reduction, if it occurred, would be at a very slow rate since 2-amino-2-thiazoline is not cytotoxic. Instead, reduction in tumor size would occur by the replacement of tumor cells by normal cells.

Each of the patients were intravenously administered 2-amino-2-thiazoline-hydrochloride, as an aqueous solution (25-75 mg/ml) at the dose of 1.5 mg/kg of body weight on a daily basis per 5 days, every three weeks (the first 5 patients), and at at dose of 3 mg/kg daily per 5 days every three weeks (the following 4 patients).

Table II shows the total dose of 2-amino-2-thiazoline hydrochloride received by each of the patients at the time the evaluation was made. None of the patients showed any toxicity and there were 4 objective responses (mor than 50% reduction of tumor size) out of the 9 cases as evaluated by the estimation of the combined area of lung metastasis and by the size of the cystoscopic lesion.

The results of this study established that 2-amino-2-thiazoline has anti-tumor activity and, therefore, is effective as a non-cytotoxic chemotherapeutic agent for the treatment of cancer.

Effect of 2-Amino-2-Thiazoline in Mouse Tumors

The effect of 2-amino-2-thiazoline on mouse tumors (leukemia 1210 and Lewis Lung carcinoma) was also tested. In these tests it was found that the drug was completely inactive in increasing the survival time of the mice inoculated with such tumors. However, it is knwon that the cells associated with those tumors are extremely undifferentiated from normal cells so as to have lost all or nearly all of the cytoskeletal system of contractile microfilaments. Such cells would be expected to be insensitive to agents which induce reverse transformation. Leukemia 1210 and Lewis Lung carcinoma are transplantable tumors and, upon transplantation, they have lost the tissue specific membrane receptors which are the target of 2-amino-2-thiazoline.

While in the foregoing experiments, 2-amino-2-thiazoline was used alone, it is within the scope of this invention to employ 2-amino-2-thiazoline or a physiologically acceptable salt thereof as part of a broader scheme of treatment for cancer in combination with one or more cytotoxic agents such as cis-platinum, 5-fluorouracil or methotrexate.

The preceding description is but illustrative of the practice of the invention and it is to be understood that other expedients may be employed by those skilled in the art without departure from the scope of the invention. Data on human tumors have been provided for bladder cancer because in a preliminary study of the biochemical affinity of 2-amino-2-thiazoline with tumor membrane receptors, the affinity with the bladder tumor receptor was very high. However, 2-amino-2-thiazoline has also shown affinity for other tumor receptors of different tissue origin and in a preliminary clinical trial, stabilization and minor remissions in tumors of various origins, are being shown. Therefore, it is claimed that 2-amino-2-thiazoline is an antitumor agent for mammalian cancer acting without toxicity by inducing reverse transformation.

## Example 1

1 kg of 2-amino-2-thiazoline hydrochloride is dissolved in 40 liters of water. Then, with continuous agitation and pH monitoring, 3,2 kg of mannitol and sufficient NaOH to bring the solution to pH 6 is added. The solution is filtered through a 0,2 μ steril filter, aseptically subdivided into vials (5 ml each), rapidly frozen at $-40^{\circ}C$ and lyophilized.

## Example 2

Tablets for oral administration containing:

| | |
|---|---|
| 2-Amino-2-thiazoline (lyophilized) | 250 mg |
| Mannitol | 300 mg |
| Conventional carrier materials | 450 mg |
| | 1000 mg |

## Example 3

1 kg of 2-amino-2-thiazoline is dissolved in 40 liters of water. To the solution aqueous hydrochloric acid is added under continuous stirring and pH monitoring until the pH is about 7. The solution is filtered, subdivided into 5 ml vials under steril conditions, frozen rapidly and lyophilized.

## Example 4

Tablets for oral administration containing:

| | |
|---|---|
| 2-Amino-2-thiazoline hydrochloride (lyophilized) | 250 mg |
| Mannitol | 350 mg |
| Conventional carrier materials | 400 mg |
| | 1000 mg |

CLAIMS:-

1. A pharmaceutical composition for the treatment of neoplastic diseases containing 2-amino-2-thiazoline or a physiologically acceptable salt thereof.

2. An injection solution for the treatment of neoplastic diseases containing 2-amino-2-thiazoline or a physiologically acceptable salt thereof.

3. An injection solution as claimed in claim 2 prepared from 2-amino-2-thiazoline or a salt thereof in lyophilized form.

4. A solid pharmaceutical composition containing 2-amino-2-thiazoline or a physiologically acceptable salt thereof in lyophilized form.

5. A lyophilized powder of 2-amino-2-thiazoline or a physiologically acceptable salt thereof.

6. The use of 2-amino-2-thiazoline and physiologically acceptable salts thereof in the treatment of neoplastic diseases.

7. The use of 2-amino-2-thiazoline and physiologically acceptable salts thereof in the treatment of cancer, particularly of bladder cancer.

8. A method of treatment neoplastic diseases in mammals which method is characterized by the administration of 2-amino-2-thiazoline or a physiologically acceptable salt thereof to the being afflicted with the diseases.

9. A method as claimed in claim 8, characterized in that cancer is treated.

10. A method as claimed in claim 9, characterized in that bladder cancer is treated.

11. A method as claimed in claim 9 or claim 10, characterized in that the compound is administered parenterally, preferably intramuscularly.

12. A method as claimed in claim 11, characterized in that there is administered an injection solution prepared from 2-amino-2-thiazoline or a physiologically acceptable salt thereof in lyophilized form.

# PARTIAL EUROPEAN SEARCH REPORT

which under Rule 45 of the European Patent Convention shall be considered, for the purposes of subsequent proceedings, as the European search report

**European Patent Office**

Application number

EP 80 30 4127

## DOCUMENTS CONSIDERED TO BE RELEVANT

| Category | Citation of document with indication, where appropriate, of relevant passages | Relevant to claim |
|---|---|---|
| X | <u>GB - A - 1 491 679</u> (A. GALLARDO)  * Pages 1,2 * | 1-7 |
| X | CHEMICAL ABSTRACTS, vol. 77, no. 15, 9th October 1972, page 16, no. 96825m Columbus, Ohio, U.S.A. M. SHINODA: "Pharmacological studies on chemical protectors against radiation. VII. Radiation protective activities of AET (S-2-aminoethylisothiuronium bromide hydrobromide) and its related compounds against z-irradiation"  & YAKUGAKU ZASSHI 1972, 92(4), 442-46  * Abstract * | 1-7 |
| X | CHEMICAL ABSTRACTS, vol. 80, no. 23, 10th June 1974, page 23, no. 128168e Columbus, Ohio, U.S.A. I. KOZAK et al.: "Metabolism and radioprotective effect of AET (S-2-aminoethylisothiuronium) and ./. | 1-7 |

**CLASSIFICATION OF THE APPLICATION (Int. Cl.³)**

A 61 K 31/425//
C 07 D 277/18

**TECHNICAL FIELDS SEARCHED (Int. Cl.³)**

C 07 D 277/18
A 61 K 31/425

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7
Claims searched incompletely:
Claims not searched: 8-12 Method for treatment
Reason for the limitation of the search: of the human or animal body by surgery or therapy (See article 52(4) of the European Patent Convention).

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| The Hague | 06-02-1981 | BRIGHENTI |

EPO Form 1505.1   06.78

| Category | Citation of document with indication, where appropriate, of relevant passages | Relevant to claim | CLASSIFICATION OF THE APPLICATION (Int. Cl.³) |
|---|---|---|---|
| | MEG (2-mercaptoethylguanidine) in organism (rat)." <br><br> & STRAHLENTHERAPIE 1973, 146(3), 369-375 <br><br> * Abstract * <br><br> -- | | |
| X | CHEMICAL ABSTRACTS, vol. 81, no. 13, 30th September 1974, page 24, no. 72624b <br> Columbus, Ohio, U.S.A. <br> G.V. DONTSOVA et al.: "Effect of aminoethylisothiuronium and 2-aminothiazoline on the level of protein thiols in bone marrow cells of mice" <br><br> & RADIOBIOLOGIYA 1974, 14(2), 285-287 <br><br> * Abstract * <br><br> -- | 1-7 | TECHNICAL FIELDS SEARCHED (Int. Cl.³) |
| X | CHEMICAL ABSTRACTS, vol. 85, no. 13, 28th March 1977, page 56, no. 87421q <br> Columbus, Ohio, U.S.A. <br> M. COLOMBO et al.: "Pharmacological properties of thiazolinobutazone (LAS 11 871). III. General pharmacology, ulcerogenic effects and acute toxicity" <br><br> & ARZNEIM.-FORSCH. 1976, 26(7), 1347-1356 <br><br> * Abstract * <br><br> ---- | 1-7 | |

DOCUMENTS CONSIDERED TO BE RELEVANT